# EUROPEAN PATENT APPLICATION

(11) **EP 4 534 055 A1**
(43) Date of publication of application: **09.04.2025**
(21) Application number: 23201226.0
(22) Date of filing: 02.10.2023
(51) Int. Cl.: A61F 13/00, A61F 13/02

(54) **A PRINTED MEDICAL DRESSING**

(71) Applicant: Mölnlycke Health Care AB, 431 21 Mölndal (SE)
(72) Inventor: MADLUNG, Peter, 416 57 GÖTEBORG (SE)
(74) Representative: Kransell & Wennborg KB

(57) **Abstract**

The present disclosure generally relates to a medical dressing (1) comprising a plurality of layers and having a first side facing the skin or wound of a patient during use, and an opposite, second side, wherein the plurality of layers comprises a backing layer (2), an adhesive skin-contact layer (3), and an absorbent pad (4) comprising one or a plurality of pad-forming layers (4a-4c); the absorbent pad (4) being arranged between the backing layer (2) and the adhesive skin-contact layer (3), wherein at least one of the plurality of layers of the medical dressing (1) comprises a UV-absorbing polymeric material, characterized in that the medical dressing (1) comprises at least one printed marking (5) visible on the second side of the medical dressing (1) and in that the printed marking (5) is a UV laser printed marking.

The present disclosure also relates to a method for printing a medical dressing (1) as defined hereinbefore.

## Description

### TECHNICAL FIELD

The present disclosure generally relates to a medical dressing comprising a plurality of layers and having a first side facing the skin or wound of a patient during use, and an opposite, second side, wherein the plurality of layers comprises a backing layer, an adhesive skin-contact layer, and an absorbent pad. The medical dressing comprises at least one printed marking being visible on the second side of the medical dressing. The present disclosure also relates to a method for producing such a medical dressing.

### BACKGROUND

Adhesive medical dressings are frequently used in wound care, both for the purpose of treating wounds and scars and for the purpose of preventing these from occurring in the first place.

An adhesive medical dressing typically comprises an adhesive skin-contact layer arranged to contact the skin or wound of a patient, as well as a top layer, often referred to as a backing layer. In many cases, particularly when the dressing is to be applied to moderate or highly exuding wounds, the dressing further comprises a wound pad arranged between the backing layer and the adhesive skin-contact layer. To protect the dressing from contamination, a release liner is applied to the adhesive skin-contact layer.

In many cases, printed markings are desirable on medical dressings. Such markings may e.g. be desired for the purpose of yielding a more visually appealing impression or for guiding the caregivers and medical staff to a correct use or application of the dressing.

To date, such usage-related information is typically provided in an instruction manual ("Instructions for use", IFU), inserted into the multi-pack in which the individual dressings are stored. However, such IFUs are generally not used or present in the actual care situation and easily become lost or dislocated.

Printed markings, such as printed instructions or graphical elements may, in some instances, be provided on the release liner of the dressing. The reason for printing on the release liner is generally to avoid colored inks inside the medical dressing. The medical dressing is to be applied to a wound or the skin of a patient, and the risk of the ink interfering with the wound or skin must generally be avoided. Printing on the release liner reduces this risk since the release liner is peeled off prior to application of the dressing onto the skin.

However, by peeling off the release liner, the printed markings (and instructions) are thereby removed from the dressing.

In view of this, there is room for improvements in the field of printed medical dressings. Hence, there is a need to provide a safe and reliable printed medical dressing, wherein the printed markings are visibly observable even when the medical dressing has been applied to the skin or wound of a patient.

### SUMMARY

In view of the above-mentioned problems, it is an object of the present disclosure to provide improvements in the field of printed medical dressings.

According to a first aspect, there is provided a medical dressing comprising a plurality of layers and having a first side facing the skin or wound of a patient during use, and an opposite, second side, wherein the plurality of layers comprises a backing layer, an adhesive skin-contact layer, and an absorbent pad comprising one or a plurality of pad-forming layers; the absorbent pad being arranged between the backing layer and the adhesive skin-contact layer, wherein at least one of the plurality of layers of the medical dressing comprises a UV-absorbing polymeric material; the medical dressing comprising at least one printed marking being visible on the second side of the medical dressing, and wherein the printed marking is a UV laser printed marking.

The present disclosure is based on the realization that a UV-laser printed marking; i.e. a printed marking formed by UV laser, provides a safe and reliable print which is not associated with the generation of any harmful substances during printing. Hence, the medical dressing can be safely applied to the skin or wound of a patient.

With a UV-laser printed marking, no ink or colorant needs to be added as is the case with conventional ink printed markings. This is beneficial to avoid the potential elution of such substances into the wound or the skin of a patient. Furthermore, no heat is generated during printing as is the case with e.g. laser printing utilizing infra-red light. The generation of heat during printing may damage the material being printed.

In addition, a UV laser printed marking is permanent and does not fade. In contrast, in conventional ink-based printing systems, there is a high risk of incomplete or faded markings.

The UV laser marked products are also resistant to post-marking damage, such as friction, heat and transportation.

Conventional ink-markings may also result in carbon dioxide emissions. Since no ink is used in the printed marking(s) of the present disclosure, such carbon dioxide emissions are eliminated.

The UV-laser printed marking may be provided by passing a beam of UV laser at a wavelength of from 320 to 380 nm (typically at about 355 nm) towards the second side of the medical dressing. The UV laser emits high-energy photons that break the polymer bonds in the UV-absorbing polymeric material of the medical dressing. When the polymer bonds are broken, the material changes colour and a visible marking is provided. The reaction of breaking the polymer bonds is a photochemical, i.e. photolytic reaction.

As mentioned hereinbefore, printing with a UV laser allows for a visible marking to be obtained without the formation of any harmful substances (see Example 2). Hence, the medical dressing can be safely applied onto the wound or the skin of a patient.

The at least one printed marking is visible on the second side of the dressing. Accordingly, the printed marking is visible when a patient is wearing the medical dressing. This is beneficial for the purpose of conveying product-identifying and usage-specific information that may guide a caregiver to a proper use or application of the medical dressing. Furthermore, such printed marking(s) may also serve the purpose of yielding a more visually appealing impression.

In exemplary embodiments, the layer comprising the UV-absorbing polymeric material is the backing layer or one of the pad-forming layer.

The backing layer is the top layer of the dressing; i.e. the layer facing a viewer (such as a caregiver).

It is, however, equally conceivable to provide the visible markings on a pad-forming layer. For example, in embodiments where the dressing comprises a transparent and non-UV-absorbing backing layer, the UV-absorbing polymeric material of a pad-forming layer of the wound pad may be the layer being printed. The printed markings are still visibly observable on the second side of the medical dressing.

In exemplary embodiments, the UV-absorbing polymeric layer further comprises a pigment.

The pigment is an additive or substance that imparts a color to the layer. This is beneficial to enhance the UV absorption properties of the polymeric material, and also to improve the contrast and focus of the UV laser beam. The visibility of the printed marking on the medical dressing is thereby enhanced.

Hence, the layer comprising the UV-absorbing polymeric material may be colored or slightly colored. The layer comprising the UV-absorbing polymeric material is typically translucent.

The pigment may be UV-absorbing.

In exemplary embodiments, the UV-absorbing polymeric material is polyurethane, polyamide, or polyethylene.

These materials are commonly used wound dressing materials, e.g. comprised in backing layers, release liners, foam wound pad layers etc.

In exemplary embodiments, the layer comprising the polymeric material is the backing layer, and wherein the UV-absorbing polymeric material is polyurethane.

For example, the backing layer may be a polyurethane film or a laminate comprising a polyurethane film.

In exemplary embodiments, the layer comprising the polymeric material may be a pad-forming layer, wherein the backing layer is transparent and non-UV-absorbing.

The pad-forming layer is typically a pad-forming layer arranged in contact with the backing layer. The pad-forming layer is thus the uppermost layer of the wound pad.

The pad-forming layer may be a polyurethane foam.

Alternatively, the pad-forming layer may be a masking layer; i.e. a layer configured to mask the wound exudate. The masking layer may be colored layer, which masks the blood and any other wound fluid in the absorbent pad, but still allows for the markings to be visible on the first side of the dressing.

The pad-forming layer may comprise a pigment. The pigment may enhance the absorption of UV laser light.

When markings are printed on a pad-forming layer, the backing layer is transparent and non-UV-absorbing. This way, the printed markings are visible on the second side of the medical dressing. The UV laser beam(s) is transported through the backing layer and is absorbed by the underlying pad-forming layer such that a printed marking is provided.

In exemplary embodiments, the at least one printed marking may be a graphical element and/or a text element.

The graphical element may be a symbol, a figure, an image, a photograph or a visual element. The graphical element may be associated with the brand or it may be used to convey information, e.g. product-identifying information or usage-specific information about the medical dressing.

The text element may e.g. be a brand name, sub-brand, a product descriptor or a size indication. The text element may also be a text message, which may be adapted to a specific patient, use or event.

In exemplary embodiments, the at least one printed marking indicates a correct use or application of the medical dressing.

As mentioned hereinbefore, usage-specific are often contained in an IFU, which, as mentioned hereinbefore, often becomes dislocated after the dressing has been applied to the skin of a patient. If such instructions are provided directly on the dressing, the caregivers are offered a clear and direct guidance of how to use and/or apply the dressing correctly.

In exemplary embodiments, the at least one printed marking is a tag being readable using an electronic device, preferably a bar code or a QR code.

The bar code or QR code may encode an internet or web address containing technical and/or usage-specific information related to the medical dressing.

Hence, information about the medical dressing may be accessed easily. To date, such information is typically present in an IFU, which is typically not accessible after the dressing has been applied to a patient.

In exemplary embodiments, the at least one printed marking may be a batch number, a production lot number, and/or a country indication.

Such markings are beneficial for regulatory purposes, anti-counterfeiting and potential product recall purposes and enables the authentication of the product. Accordingly, each dressing may be tracked and traced, e.g. back to the earliest steps of manufacturing.

The provision of a printed country indication is also useful for preventing illegal imports; i.e. branded products being imported into a market and sold there without the trademark owner's consent in that market or country. The country indication may be the country of manufacturing or the country of commercialization.

It is also conceivable that the information about the batch number, production lot number or country indication is encoded by the QR code or bar code.

According to another aspect, there is provided a method for printing a medical dressing comprising:
a) providing a medical dressing comprising a plurality of layers and having a first side facing the skin or wound of a patient during use, and an opposite, second side, wherein the plurality of layers comprises a backing layer, an adhesive skin-contact layer, and an absorbent pad comprising one or a plurality of pad-forming layers; the absorbent pad being arranged between the backing layer and the adhesive skin-contact layer,
b) printing the medical dressing by passing one or a plurality of UV laser beams towards the second side of the medical dressing such that the UV laser beam(s) form(s) at least one printed marking on the medical dressing.

The method of the present disclosure is advantageous since the printing can be precisely controlled and the marking(s) can be tailored to specific and desired positions on the medical dressing. In contrast, conventional ink printing typically suffers from problems with skewing and the risk for the print to become altered during subsequent process steps. Thus, the method of the present disclosure offers a controlled and tailored means for printing.

In the first step (step a), a medical dressing is provided by conventional dressing manufacturing techniques. The medical dressing comprises a plurality of layers, and at least one of these layers comprises a UV-absorbing polymeric material.

Step b) of printing is accomplished by passing one or a plurality of UV laser beams towards the second side of the medical dressing. The layer comprising the UV-absorbing polymeric material will absorb the UV laser light such that at least one printed marking is formed on the medical dressing.

The step b) of printing may be performed as an in-line step in the manufacturing process of the medical dressing.

The method may further comprise a step of
a') arranging the medical dressing in a package comprising at least a front layer and a back layer, wherein at least a portion of the front layer is transparent and non-UV-absorbing, and wherein the medical dressing is arranged in the package such that the second side of the dressing is visible through the transparent and non-UV-absorbing portion of the front layer, wherein step a') is performed prior to step b), and wherein the step b) of printing is performed by passing one or a plurality of UV laser beams through the transparent and non-UV-absorbing portion of the front layer.

Accordingly, the step a') of printing is accomplished with the dressing remaining inside the package.

The medical dressing is arranged such that the second side of the dressing is visible through the transparent portion of the front layer. Hence, the dressing is arranged such that its top layer, i.e. backing layer, is visible through the package.

The UV laser beam(s) passes through the transparent and non-UV-absorbing portion of the front layer and is absorbed by the UV-absorbing polymeric material in the medical dressing. The UV-absorbing polymeric material undergoes a photolytic reaction such that the polymer bonds break, and a visible marking is provided on the medical dressing. The transparent front layer of the package does not absorb UV light, and therefore remains unaffected by the UV laser beam(s).

Accordingly, the dressing is protected from potential contaminants or substances during the printing step.

The method may further comprise a step of sterilizing the package and the medical dressing (1) prior to the step b) of printing the medical dressing.

Hence, the printed markings may be provided on the sterilized dressing without altering the sterility of the dressing or the sterile interior of the package. Furthermore, the sterilization process does not negatively impact the ability of the medical dressing to become printed.

The package and the dressing are typically sterilized simultaneously.

The package is preferably sealed prior to the step b) of printing.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 illustrates a medical dressing comprising a printed marking on the backing layer according to an exemplary embodiment of the present disclosure. In figure 1, the medical dressing is arranged in a package, and portions of the dressing and the package have been cut out to illustrate the various layers.
Figure 2 illustrates a top-view of the medical dressing, provided with printed markings in the form of a QR code and a text message, according to an exemplary embodiment of the present disclosure.
Figure 3 illustrates a top view of a medical dressing comprising printed markings in the form of a cutting guide indicating how the dressing should be cut, as seen from the first side of the medical dressing.
Figure 4 illustrates a patient wearing the cut out dressing pieces when the medical dressing of figure 3 has been cut along the printed cutting guide lines.
Figure 5a schematically illustrates a method for printing a medical dressing according to an exemplary embodiment of the present disclosure.
Figure 5b illustrates a cross-sectional view along the line A-A in figure 1a.
Figure 6 schematically illustrates the UV laser printer utilized in Example 1 and Example 2.
Figures 7a, 7b and 7c illustrate photographs of the printed wound dressings of Example 1.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

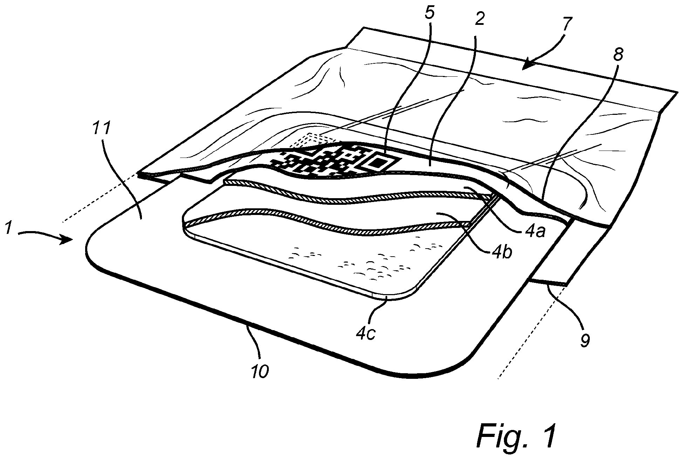

A medical dressing is schematically illustrated in figure 1. In this figure, the medical dressing 1 is arranged in a package 7.

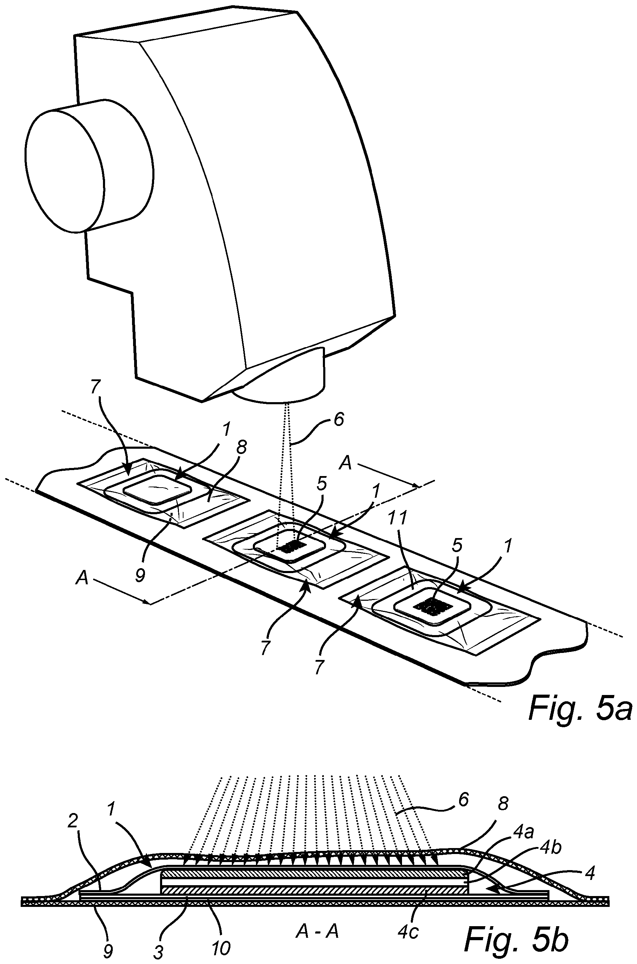

As best illustrated in figure 1 and in figure 5b, the medical dressing 1 comprises a plurality of layers and has a first side facing the skin or wound of a patient during use, and an opposite, second side, wherein the plurality of layers comprises a backing layer 2, an adhesive skin-contact layer 3, and an absorbent pad 4 comprising one or a plurality of pad-forming layers 4a-4c; the absorbent pad 4 being arranged between the backing layer 2 and the adhesive skin-contact layer 3, wherein at least one of the plurality of layers of the medical dressing 1 comprises a UV-absorbing polymeric material; the medical dressing 1 comprising at least one printed marking 5 being visible on the second side of the medical dressing 1 and wherein the printed marking 5 is an UV laser printed marking.

As used herein, the term "UV laser printed marking" means a printed marking formed by one or a plurality of UV laser beams. The wavelength of the UV laser is typically in the range of from 320 to 380 nm, preferably at or about 355 nm.

The "UV-absorbing polymeric material" absorbs the UV light, and a photochemical reaction takes place, which breaks the polymeric bonds, thereby yielding a visible marking which is visible on the second side of the dressing.

The UV-absorbing polymeric material is comprised in one of the layers of the medical dressing 1.

Typically, the layer comprising the UV-absorbing polymeric material is the backing layer or one of the pad-forming layers.

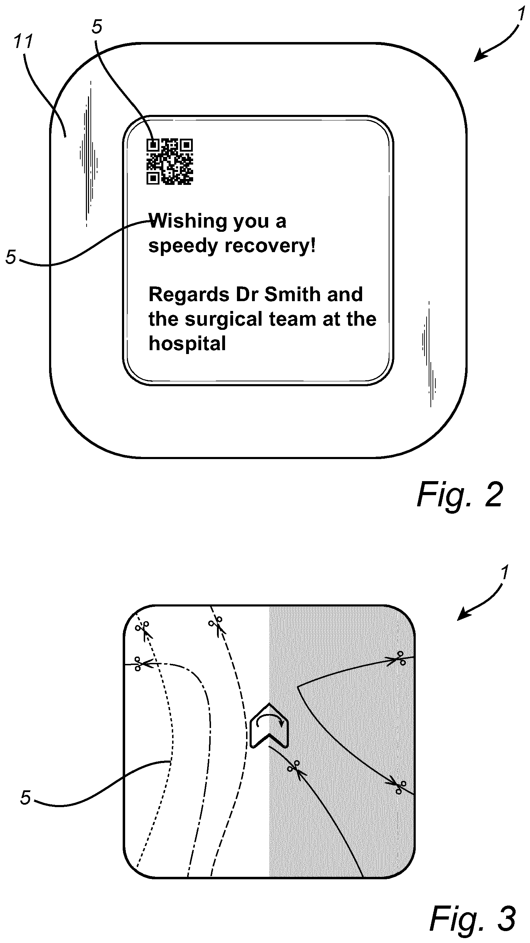

The backing layer 2, absorbent pad 4 and the adhesive skin-contact layer 3 may be co-extensive; i.e. they may have the same surface area. Such a configuration is illustrated in figure 3.

Alternatively, the backing layer 2 and the adhesive skin-contact layer 3 are configured to extend around the contour of the absorbent pad 4 to form a border portion 11. Such a medical dressing is typically referred to as a "border dressing". A border dressing is illustrated in figure 1, figure 2 and figures 5a-b.

The medical dressing 1 typically further comprises a release liner 10 detachably attached to the adhesive skin-contact layer 3. The release liner protects the skin-contact layer (and the dressing) from contamination prior to use. The release liner 10 is co-extensive in length and in width with the adhesive skin-contact layer 3.

Accordingly, when the dressing is arranged in a package 7, as illustrated in figure 1 and figures 5a-b, the release liner 10 is attached to the adhesive skin-contact layer 3. The medical dressing 1 is arranged in the package 7 such that the backing layer 2 of the medical dressing 1 faces the front layer 8 of the package 7 (and is viewable through the transparent portion of the front layer 8), and the release liner 10 faces the back layer 9 of the package 7, as best illustrated in figure 5b.

The "backing layer" is the top layer of the dressing; i.e. the outermost layer of the dressing. The backing layer is desirably a thin and pliable layer such that it can stretch and conform to the movement of a wearer. The backing layer may comprise the UV-absorbing polymeric material. The backing layer may be a polymeric film. Suitably, the backing layer comprises a polyurethane film. The backing layer may also be a laminate of a nonwoven layer and at least one polyurethane film. For example, the backing layer may, in some embodiments be a laminate of at least one non-UV-absorbing layer and a UV-absorbing layer.

The thickness of the backing layer may be in the range of from 15 to 50 µm, preferably from 20 to 40 µm.

In exemplary embodiments, the backing layer is translucent and/or colored.

The "adhesive skin-contact layer" is configured to detachably adhere the dressing to a dermal surface. In other words, the adhesive skin-contact layer is configured to contact the skin or the wound of a wearer. This layer may also be referred to as a "wound contact layer".

The adhesive skin-contact layer preferably comprises a silicone-based adhesive; e.g. a silicone gel. An adhesive skin-contact layer comprising a silicone gel is skin-friendly and easy to remove without causing trauma. It is sufficiently adherent to skin such that the dressing stays in place, but is configured to maintain its adherence with repeated removal and re-application.

The adhesive skin-contact layer comprising the silicone-based adhesive may be provided as a coating on the absorbent pad, e.g. on the lowermost pad-forming layer of the absorbent pad. The adhesive skin-contact layer may also comprise two layers. For example, the adhesive skin-contact layer may comprise a polymer-based film and a silicone gel layer, wherein the silicone gel layer is configured to contact the skin of a wearer during use.

Such an adhesive skin-contact layer is suitably used in embodiments where the dressing is a border dressing.

The polymer-based film may be a breathable film and may comprise e.g. polyethylene, polyamide, polyester or polyurethane. Preferably, the polymer-based film comprises polyurethane. The thickness of the polyurethane film may be from 15 to 100 µm, e.g. from 20 to 80 µm, preferably from 20 to 60 µm.

Examples of suitable silicone gels for use in the adhesive skin-contact layer 102 and/or in the silicone gel layer 102b include the two component RTV systems, such as Q72218 (Dow Corning), and SilGel 612 (Wacker Chemie AG) mentioned herein, as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the adhesive skin-contact layer is typically at least 20 µm. The thickness of the adhesive skin-contact layer may be from 100 to 200 µm.

The adhesive skin-contact layer 3 may be perforated or non-perforated.

In embodiments where the dressing comprises a border portion 11, the adhesive skin-contact layer 3 typically comprises a plurality of perforations, at least in the area underlying the absorbent pad 4.

As illustrated in figure 1 and figure 5b, the absorbent pad 4 may comprise one or a plurality of pad-forming layers 4a-c.

The absorbent pad 4 may e.g. comprise an absorbent polyurethane foam layer. It is also conceivable that the absorbent pad 4 consists of an absorbent polyurethane foam layer.

The absorbent pad 4 may further comprise a superabsorbent layer; i.e. a layer comprising a superabsorbent material. The superabsorbent material may be superabsorbent polymer (SAP) particles or superabsorbent fibres (SAF). The superabsorbent material is capable of handling large amounts of wound exudate.

The absorbent pad 4 may also comprise a liquid distribution layer. The liquid distribution layer may comprise any material having the ability to distribute the exudate in an efficient manner. For example, the liquid distribution layer may comprise a nonwoven material.

Such a layered pad construction prevents accumulation of body liquids close to the skin and improves the overall liquid handling of the medical dressing.

In figure 1 and figure 5b, the medical dressing may e.g. comprise a superabsorbent layer 4a, a liquid distribution layer 4b, and polyurethane foam layer 4c.

Typically, the layer comprising the UV-absorbing polymeric material is the backing layer 2 or a pad-forming layer 4a-c. In embodiments where the layer comprising the UV-absorbing material is a pad-forming layer 4a-c, the UV-absorbing polymeric material is comprised in the uppermost layer of the absorbent pad.

The layer comprising the UV-absorbing polymeric material may further comprise a pigment.

The present disclosure is not limited to the use of a specific pigment, as long as the pigment imparts a color to the layer. The layer comprising the UV-absorbing polymeric material may e.g. be beige or white. Other colors are also conceivable.

The pigment may be a UV-absorbing pigment.

The UV-absorbing polymeric material may be polyurethane, polyamide, or polyethylene.

These materials are suitably used in the various layers of the dressing. A polyurethane, polyamide or a polyethylene layer may e.g. be used as the backing layer or as a sub-layer of an adhesive skin-contact layer. A pad-forming layer comprising a polyurethane foam is also suitably used. A polyurethane foam is beneficial for the purpose of absorbing large amounts of wound exudate.

The layer comprising the UV-absorbing polymeric material may be the backing layer 2, and wherein the UV-absorbing polymeric material is polyurethane. In figure 1, printed markings 5 have been provided on the backing layer 2 which comprises the UV-absorbing polymeric material.

Typically, the backing layer 2 is a polyurethane layer.

It is equally conceivable that the layer comprising the UV-absorbing polymeric material is a pad-forming layer, wherein the backing layer is transparent and non-UV-absorbing.

In embodiments where the pad-forming layer 4a-c comprises a UV-absorbing material, the backing layer is non-UV-absorbing and transparent. Hence, the backing layer will be unaffected by the UV laser beam.

For example, the medical dressing schematically illustrated in figure 3 typically comprises one polyurethane foam layer and a transparent backing layer being heat-laminated onto the polyurethane foam layer. The printed markings 5 may thus be printed on the polyurethane foam layer.

The at least one printed marking 5 may be a graphical element and/or a text element.

As used herein, the term "graphical element" means a symbol, a figure, an image, a photograph, a logotype, a shape, a pattern, and/or a visual element. The graphical element may be associated with the brand, or it may be used to convey information, e.g. product-identifying information or usage-specific about the medical dressing 1.

The "text element" may be a brand name, sub-brand, a product descriptor, an alphabet letter, a number, and/or a size indication. The text element may also be a text message, which may be adapted to a specific patient, use or event.

A dressing comprising a printed marking in the form of a text message is illustrated in figure 2.

The flexible printing method of the present disclosure allows for various tailored and event-specific markings to be provided on the medical dressing 1.

The at least one printed marking 5 may indicate a correct use or application of the medical dressing 1.

There are various scenarios where such printed instructions are beneficial. For example, if the dressing has a "complex" shape with e.g. a plurality of protruding border portion, which is often the case with heel dressings (and other dressings that are to be applied onto contoured body parts), then the printed markings may indicate a correct application of the dressing onto that body part.

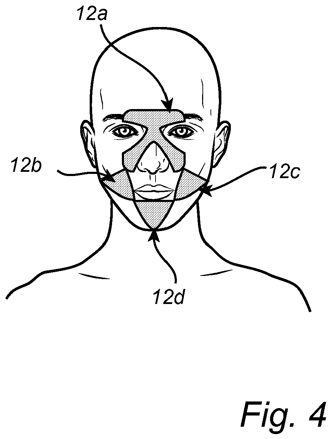

Furthermore, as illustrated in figure 3, the medical dressing 1 may comprise printed markings 5 in the form of cutting guide lines. The cutting guide lines indicate to a caregiver how to cut the dressing into various dressing pieces that may be used underneath a medical device. In figure 4, a patient wearing the dressing pieces 12a-d is illustrated, and this dressing piece arrangement may e.g. be used underneath a CPAP mask.

The at least one printed marking 5 may be a tag being readable using an electronic device, preferably a bar code or a QR code.

A medical dressing 1 comprising a printed QR code is e.g. illustrated in figure 2 and in figures 5a-b.

The bar code or QR code may encode an internet or web address containing technical and/or usage-specific information related to the medical dressing.

The at least one printed marking 5 may be a batch number, a production lot number, or a country indication.

Such markings are beneficial for regulatory purposes, anti-counterfeiting and potential product recall purposes and enables the authentication of the product. Accordingly, each dressing may be tracked and traced, e.g. back to the earliest steps of manufacturing.

To date, e.g. the lot number may be provided on the package comprising the dressing. However, the package is often discarded after application of the medical dressing. This renders the traceability of the dressing more difficult since a potential "problem" may be detected at a later stage.

It is also conceivable that the printed markings may comprise the lot sequence number; i.e. the exact sequence number in a lot of dressings. This is useful to enable an exact tracing of a potential error in the manufacturing chain.

A printed country indication is also useful for preventing illegal imports, which is a common problem in the field of advanced medical dressings. The country indication may be the country of manufacturing or the country of commercialization.

It is also conceivable that information about the batch number, production lot number or country indication is encoded by the QR code or bar code.

According to another aspect, and as illustrated in figures 5a and 5b, there is provided a method for printing a medical dressing comprising:
a) providing a medical dressing 1 comprising a plurality of layers and having a first side facing the skin or wound of a patient during use, and an opposite, second side, wherein the medical dressing 1 comprises a backing layer 2, an adhesive skin-contact layer 3, and an absorbent pad 4 comprising one or a plurality of pad-forming layers 4a-c; the absorbent pad 4 being arranged between the backing layer 2 and the adhesive skin-contact layer 3, wherein at least one of the plurality of layers of the medical dressing 1 comprises a UV-absorbing polymeric material,
b) printing the medical dressing 1 by passing one or a plurality of UV laser beams 6 towards the second side of the medical dressing 1 such that the UV laser beam(s) form(s) 6 at least one printed marking 5 on the medical dressing 1.

The step a) of providing a medical dressing may be performed by techniques and manufacturing processes well known to the skilled person.

The various layers of the dressing may be attached by adhesion or lamination.

Step b) of printing the medical dressing 1 is performed by passing one or a plurality of UV laser beams 6 towards the second side of the medical dressing 1. The visible marking 5 is formed on the layer comprising the UV-absorbing polymeric material in the medical dressing 1. The markings 5 are visible on the second side of the medical dressing 1.

The polymeric material comprised in one of the layers of the dressing absorbs the UV light, and a photochemical reaction takes place, which breaks the polymeric bonds, thereby yielding a visible marking which is visible on the first side of the dressing.

As demonstrated by Example 2, no harmful biproducts are formed during printing. Accordingly, the printed medical dressing 1 can be safely applied to the wound and/or skin of a patient.

The present disclosure is not limited to a specific UV laser equipment, but any equipment known to the skilled person may be used.

The wavelength of the UV laser is typically in the range of from 320 to 380 nm, preferably at or about 355 nm.

The method is advantageous since the printing can be precisely controlled and the marking(s) can be tailored to specific and desired positions on the medical dressing. In contrast, conventional ink printing typically suffers from problems with skewing and the risk for the print to become altered during subsequent process steps. Thus, the method of the present disclosure offers a controlled and tailored means for printing.

Furthermore, interruptions caused by e.g. ink ribbon and thermal head replacements (or cleaning) are eliminated. Accordingly, a faster printing process is provided, and more products may be produced in the same amount of time.

The UV-absorbing polymeric material absorbs the UV laser light strongly, which causes the intermolecular or interatomic bonds to break, thereby resulting in a visible marking 5.

The method may further comprise a step of:
a') arranging the medical dressing 1 in a package 7 comprising at least a front layer 8 and a back layer 9, wherein at least a portion of the front layer 8 is transparent and non-UV-absorbing, and wherein the medical dressing 1 is arranged in the package 7 such that the second side of the medical dressing 1 is visible through the transparent and non-UV-absorbing portion of the front layer 8 , wherein step a') is performed prior to step b), and wherein the step b) of printing is performed by passing the one or a plurality of UV laser beams 6 through the transparent and non-UV-absorbing portion of the front layer 8.

Figures 5a and 5b illustrates a method where printing is accomplished through the transparent front layer 8 of the package 7.

The transparent, and non-UV-absorbing portion of the front layer 8 remains unaffected by the UV laser beam 6, which is transported through the front layer 8.

The printed marking is visible through the transparent portion of the front layer 8 of the package 7 during storage and transport.

An advantage with printing with the dressing remaining inside the package is that the dressing is protected from potential contaminants or substances during the printing step.

Typically, the transparent portion of the front layer 8 is large to improve the flexibility of the printing process and to minimizes errors during printing.

Typically, at least 70%, preferably at least 80% of the front layer 8 is transparent and non-UV-absorbing.

Accordingly, at least 70%, preferably at least 80% of the surface area of the front layer 8 is transparent and non-UV-absorbing.

Preferably, the entire front layer 8 is transparent and non-UV-absorbing. In figure 5a, the entire front layer 8 is transparent.

The package 7, in which the medical dressing 1 is arranged (step a'), is typically a pouch comprising a front layer and a back layer which are sealed together at the peripheral side edges. When the medical dressing 1 is arranged in the package 7; i.e. pouch, at least one side edge is open to allow entry of the medical dressing 1. When the medical dressing 1 is to be used and taken out of the package 7, the sealed side edge may be torn open (see figure 1).

The package 7 may be a pouch comprising a front layer 8 and a back layer 9, as illustrated in figure 1, figure 5a, and figure 5b.

The back layer 9 of the package is not limited to a specific material. For example, the back layer 9 may comprise paper.

The transparent portion of the front layer 8 is not limited to a specific material as long as the material is transparent and non-UV-absorbing. For example, the transparent portion of the front layer 8 may be a laminate comprising a plurality of layers. For example, the transparent portion of the front layer may comprise polyethylene terephthalate (PET). The thickness of the transparent front layer may be from 40 µm to 200 µm.

The method typically comprises a step of sealing the front layer 8 and the back layer 9 before the step b) of printing the medical dressing 1.

The front layer 8 and back layer 9 are defined by peripheral side edges. The peripheral side edges of the front layer 8 may be sealed to the peripheral side edges of the back layer 9 before the step c) of printing the medical dressing 1.

The method may further comprise a step of sterilizing the package 7 and the medical dressing 1 prior to the step b) of printing the medical dressing 1.

Typically, the package 7 and the medical dressing 1 are sterilized simultaneously.

Preferably, the package 7 and the medical dressing 1 are sterilized by Ethylene oxide (EtO) sterilization. This sterilization method allows the medical dressing to be sterilized in the final package, as ethylene oxide permeates the sealed films and cartons used to package the dressings.

### Example 1

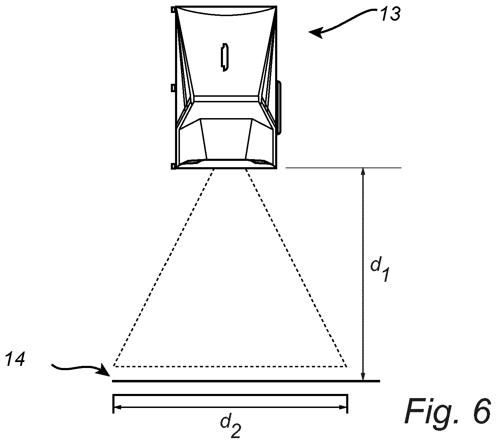

Dressing samples were marked with a 3-Axis UV laser marker (MD-U1020C from Keyence). The UV laser marker is schematically illustrated in figure 6 (denoted 13). The wavelength of the UV laser light was 355 nm. The 3-Axis UV laser marker utilizes an automatic focus functionality allowing products to be placed on a reference surface (denoted 14 in figure 6) and the MD-U unit identifies the distance, d1, to the surface to be marked and adjusts both the laser focus distance and power automatically.

The distance, d1, in figure 6 may be 300 mm ± 21 mm. The distance, d2, may be 330 mm.

The dressing samples tested were:
Dressing A: Mepilex^{®} Border Flex (Mölnlycke Health Care), which comprised a beige coloured polyurethane backing layer (thickness: 20 µm), a wound pad and a wound contact layer comprising a silicone-based adhesive.
Dressing B: Mepilex^{®} Lite foam dressing (Mölnlycke Health Care) comprising a beige colored polyurethane foam layer, a wound contact layer comprising a silicone-based adhesive, and a polyurethane backing layer heat-laminated onto the polyurethane foam layer.

Dressing A was arranged in a sealed sterile pouch prior to printing. The sterile pouch contained a back layer (paper) and a transparent front layer (a non-UV-absorbing laminate comprising polyethylene terephthalate (PET)). The printing was performed through the transparent front layer with the backing layer of the dressing being visible through the transparent front layer and facing the UV laser. A metal weight was used to secure the dressing in position during printing.

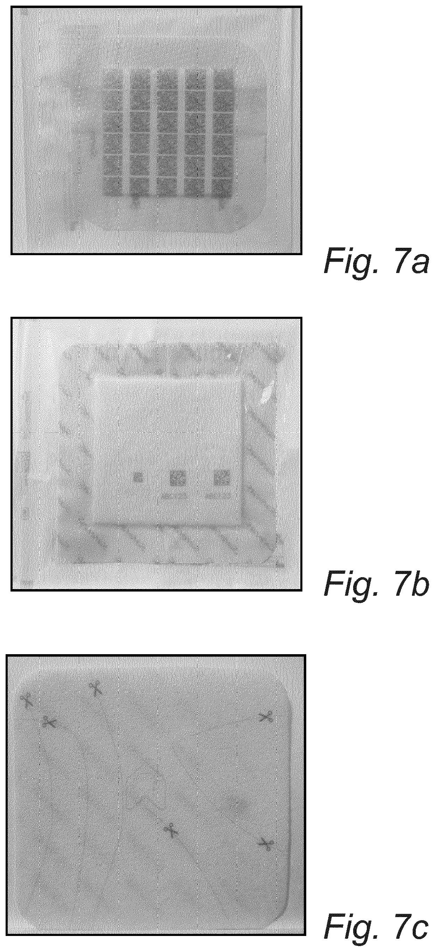

The UV laser marking process was activated and the polyurethane backing layer was marked through the transparent front layer with a 2D code based on 34 variables, 0107332430446527172305281020267731. The printed dressing is illustrated in figure 7a. The dressing illustrated in figure 7b was printed in the same manner, and also with a string of characters and variables ABC123.

Information about the contents of 2D code and the string was inserted to the computer software connected to the MD-U unit.

Dressing B was printed outside the sterile pouch, i.e. directly on the side of the dressing facing the UV laser (i.e. the side facing away from the wound during use). The dressing was arranged on the reference surface (denoted 14 in figure 6). Dressing B was marked with cutting guide markings, as illustrated in figure 7c. The cutting guide markings were defined using a 2D CAD software and the CAD file was inserted into the computer software connected to the MD-U unit.

As illustrated in figures 7a-c, clear and visible markings were provided on the dressing samples.

### Example 2

Sterilized dressing samples were made for laboratory tests to investigate if any harmful substances were generated during UV laser printing.

The dressing samples tested were Mepilex^{®} Border Flex Lite (Mölnlycke Health Care), which comprised a beige coloured polyurethane backing layer (thickness: 20 µm), a wound pad and a wound contact layer comprising a silicone-based adhesive.

Printing was performed in the manner described in Example 1; i.e. while the dressing samples were arranged in the sterile package. This method was used to prevent the samples from becoming contaminated by any external substances and, by this, knowing that all identified substances found on the UV laser marked samples (that are not identified on the tested reference non UV laser marked samples), are a result of the UV laser marking of the polyurethane backing layer.

The polyurethane backing layer (60*60 mm) was separated from the rest of the product and was weighed and extracted in dichloromethane in a sonic bath. The extract was analyzed by Gas Chromatography-Mass Spectrometry (GC-MS) where the detected compounds were identified by NIST library of mass spectra. The concentrations were estimated in decane equivalents using external standard.

The results from the characterization of organic compounds are shown in Table 1 hereinbelow.

**Table 1: Characterization of organic compounds in UV laser printed product compared to reference**

| Compound | CAS | Printed (µg/cm²) | Reference (µg/cm²) |
|---|---|---|---|
| p,p'-Methylenebis[phenyl isocyanate] (MDI) | 101-68-8 | 2.2 | 2.3 |
| Irganox 1076 | 2082-79-3 | 0.5 | 0.4 |

To summarize, no harmful substances were generated during the UV laser printing.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A medical dressing (1) comprising a plurality of layers and having a first side facing the skin or wound of a patient during use, and an opposite, second side, wherein said plurality of layers comprises at least a backing layer (2), an adhesive skin-contact layer (3), and an absorbent pad (4) comprising one or a plurality of pad-forming layers (4a-4c); said absorbent pad (4) being arranged between said backing layer (2) and said adhesive skin-contact layer (3), wherein at least one of said plurality of layers of said medical dressing (1) comprises a UV-absorbing polymeric material, **characterized in that** said medical dressing (1) comprises at least one printed marking (5) being visible on said second side of said medical dressing (1) and **in that** said printed marking (5) is a UV laser printed marking.

2. The medical dressing (1) according to claim 1, wherein said layer comprising said UV-absorbing polymeric material is said backing layer (2) or one of said pad-forming layers.

3. The medical dressing (1) according to claim 1 or claim 2, wherein said layer comprising said UV-absorbing polymeric material further comprises a pigment.

4. The medical dressing (1) according to any one of claims 1-3, wherein said UV-absorbing polymeric material is polyurethane, polyamide or polyethylene.

5. The medical dressing (1) according to claim any one of the preceding claims, wherein said layer comprising said UV-absorbing polymeric material is said backing layer (2), and wherein said backing layer (2) comprises a polyurethane film.

6. The medical dressing (1) according to any one of the preceding claims, wherein said layer comprising said UV-absorbing polymeric layer is a pad-forming layer (4a-c), and wherein said backing layer (2) is transparent and non-UV-absorbing.

7. The medical dressing (1) according to any one of the preceding claims, wherein said at least one printed marking (5) is a graphical element and/or a text element.

8. The medical dressing (1) according to any one of the preceding claims, wherein said at least one printed marking (5) indicates a correct use or application of said medical dressing (1).

9. The medical dressing (1) according to any one of the preceding claims, wherein said at least one printed marking (5) is a tag being readable using an electronic device, preferably a bar code or a QR code.

10. The medical dressing (1) according to any one of the preceding claims, wherein said at least one printed marking (5) is a batch number, a production lot number, or a country indication.

11. A method for printing a medical dressing comprising:
a) providing a medical dressing (1) comprising a plurality of layers and having a first side facing the skin or wound of a patient during use, and an opposite, second side, wherein said plurality of layers comprises at least a backing layer (2), an adhesive skin-contact layer (3), and an absorbent pad (4) comprising one or a plurality of pad-forming layers (4a-c); said absorbent pad (4) being arranged between the backing layer (2) and the adhesive skin-contact layer (3), wherein at least one of said plurality of layers of said medical dressing (1) comprises a UV-absorbing polymeric material,
b) printing said medical dressing (1) by passing one or a plurality of UV laser beams (6) towards the second side of the medical dressing (1) such that said UV laser beam(s) form(s) (6) at least one printed marking (5) on said medical dressing (1).

12. The method according to claim 11, further comprising a step of
a') arranging said medical dressing (1) in a package (7) comprising at least a front layer (8) and a back layer (9), wherein at least a portion of said front layer (8) is transparent and non-UV-absorbing, and wherein said medical dressing (1) is arranged in said package (7) such that the second side of said medical dressing (1) is visible through said transparent and non-UV-absorbing portion of said front layer (8), wherein said step a') is performed prior to said step b), and wherein said step b) of printing is performed by passing said one or a plurality of UV laser beams (6) through said transparent and non-UV-absorbing portion of said front layer (8).

13. The method according to claim 12, further comprising a step of sterilizing said package (7) and said medical dressing (1) prior to said step b) of printing said medical dressing.
